# EUROPEAN PATENT APPLICATION

(11) **EP 3 081 260 A1**
(43) Date of publication of application: **19.10.2016**
(21) Application number: 16151319.7
(22) Date of filing: 31.08.2007
(51) Int. Cl.: A61N 1/40, A61M 21/00, A61N 1/36

(54) **MOTION SICKNESS DEVICE**

(30) Priority: 01.09.2006 AU 2006904805
(62) Divisional of application: 07800228.4
(71) Applicant: GSMO Pty Ltd, Reservoir VIC 3073 (AU)
(72) Inventor: Braun, George Robert, Reservoir, Victoria 3037 (AU)
(74) Representative: Hamilton, Alistair

(57) **Abstract**

An electrically powered device (160) including an insertion component (165) for insertion into the ear cavity of a user, the device (160) including at least one inductor (170) operatively connected to a control circuit (175), the control circuit (175) providing electrical energy to the at least one inductor (170) wherein the insertion component (165) includes the at least one Inductor (170) such that when In use, the at least one inductor (170) is in close proximity to the inner ear of the user.

## Description

### field of Invention

The invention relates generally to a method and apparatus for alleviating the sensation commonly referred to as "motion sickness".

### Background of the Invention

The sensation of motion sickness is an unpleasant sensation of nausea and dizziness that some people experience when riding in a moving vehicle. Motion sickness can be brought on by travelling in cars, boats, submarines, aeroplanes, trains, by riding amusement rides that spin, and even when using a swing at a playground. Motion sickness is also known as travel sickness. Other popular terms depend on the mode of transport for example, airsickness, carsickness and seasickness.

The sensation of motion sickness can lead to feelings of discomfort and nausea, and in severe cases can lead to vomiting. Frequent vomiting can lead to dehydration and low blood pressure, so it is important for sufferers to seek prompt medical attention if they are severely affected.

Inside the inner ear is a series of canals filled with fluid generally referred to as the labyrinth, which includes semicircular shaped canals. Three semicircular canals are arranged at different angles. When the head is moved, the rolling of the fluid inside these canals tells the brain exactly how far, how fast and in what direction the head is' moving.

Information from these canals is passed along to the brain via the vestibular nerve, which lies next to the cochlear nerve. If the brain is aware of the position of the head, it can determine the position of the rest of the body.

The brain also relies on information from the eyes and from the muscles themselves (called 'muscle sense' or kinaesthesia). The brain uses the inner ear, the eyes and muscles to determine the position of the body at all times.

The sensation of motion sickness appears to arise as a result of a clash of sensory information (eg. when the eyes and muscles are indicating that the body is relatively stationary from observing and resting in the vehicle, whilst the fluid in the semicircular canals indicates that the head is moving as a result of movement of the vehicle).

There are various steps that can be taken to prevent or reduce the effects of motion sickness, such as eliminating the intake of alcohol 24 hours before travelling, avoiding cating and drinking whilst travelling, and avoiding strong smells or furnes/smoke as each of these can have the effect of exacerbating symptoms.

Whilst there arc medications available, they either act to calm the nerves of the inner ear or soothe the part of the brain that induces vomiting. However, all medications arc preventative and not curative. As a result, they are only effective if taken before the onset of motion sickness and can cause drowsiness as a side effect. Of course, a mild suffered could conceivably take medication and suffer side effects although the journey they embark upon may not have caused them to suffer motion sickness.

Depending upon the sensitivity of the particular individual, some people can experience the sensation of motion sickness with virtually any form of travel motion. In any event, despite the sensitivity of any particular individual, the condition is particularly noticeable during travel across water. As a result, in order to avoid the sensation of motion sickness, many people are inclined to avoid certain types of travel and in particular, water based travel.

In addition to restricting people with respect to their travel options, avoiding water-based travel also prevents many people from enjoying water sports or leisure activities involving water-borne vessels, such as fishing, water skiing and cruising.

Past attempts to alleviate motion sickness have substantially failed or cause side effects or require restricted activity either before or during travel. Accordingly, there is a need for an apparatus and method to alleviate the sensation of motion sickness that does not include the restrictions or inconvenience associated with existing remedies or methods.

Any discussion of documents, acts, materials, devices, articles or the like, which has been included in the present specification is solely for the purpose of providing a context for the present invention. It is not to be taken as an admission that any or all of these matters form part of the prior art base or were common general knowledge in the field of the invention, as it existed before the priority date of any of the claims herein.

### Summary of the invention

In one aspect, the present invention provides an electrically powered device including an insertion component for insertion into the car cavity of a user, the device including at least one inductor operatively connected to a control circuit, the control circuit providing electrical energy to the at least one inductor wherein the insertion component includes the at least one inductor such that when in use, the at least one inductor is in close proximity to the inner ear of the user.

In one exemplary embodiment, the inductor in the insertion component is pulsed with electrical energy every few seconds, thus causing a pulse of electromagnetic radiation to be emitted from the inductor and impinging upon the inner car region of the user.

In another exemplary embodiment, the rate/amplitude of the electrical energy is controllable by the user with an adjustment means. In this particular embodiment, the control circuit is operatively connected to a switch, or other user operable interface, to enable the user to adjust the amplitude/frequency of the electrical energy. The control of the amplitude/frequency of the electrical energy may occur remotely from the electrically powered device. The remote control device could provide control signals either by infra-red or by a radio frequency link to the electrically powered device.

Of course, any number of pulses of electrical energy over a period of time could be provided according to the effectiveness of the stimulation for any particular individual. Further, the profile (or waveform) of the electrical energy provided to the inductor could be varied to best suit individual requirements. For example, the provision of pulses could be periodic, a-periodic, or any combination thereof.

In an exemplary embodiment, a range of pre-programmed profiles are provided that are selectable by the user. Once an effective "pre-programmed" profile is located, the user may then further tailor the profile in an attempt to further increase the effectiveness. Alternatively, the user can develop their own custom profile in an attempt to develop the most effective profile for their individual requirements.

In an exemplary embodiment, the user may adjust the amplitude and/or frequency of the electrical energy provided to the inductor whilst travelling. This enables a user to accommodate any varying intensity of the sensation of motion sickness and adjust the profile in an attempt to optimally alleviate the sensation of motion sickness during travel.

The insertion component may be formed as a moulded rubber or plastic component that sits comfortably in the ear cavity of a user and contains at least one inductor within the moulding. Of course, many different types of materials can be used to form the insertion component but preferably, a material is selected that does not interfere with the emission of the electromagnetic emissions from the inductor when in use.

There are many variations possible for the specific arrangement of the insertion component and in one exemplary embodiment, the insertion component is configured to attach to the outer ear of a user, in order to provide further security with respect to attachment and/or reducing the requirement for a relatively snug fit of the insertion component with the ear cavity of the user.

The characteristics of the inductor may vary and in one exemplary embodiment, the characteristics of the inductor are selected in combination with the characteristics of the electrical energy pulse that is passed through the inductor, such that the overall result is an electromagnetic pulse that provides stimulation to the inner ear region of the user.

Electrical energy for the device may be sourced from self-contained batteries that are permanently connected to the control circuit and inductor arrangement. In this particular embodiment, the batteries may be rechargeable batteries and the electrically powered device may include the necessary circuitry to enable the batteries to be recharged whilst located within the electrically powered device. However, many other suitable sources of electrical energy may be used, such as mains power or an external battery, such as the battery of a motor or water based vehicle. Similarly, connection to an external source of electrical energy may be effected in order to recharge the batteries contained within the electrically powered device.

In one exemplary embodiment, the insertion component includes at least one inductor and an audio transducer for converting electrical energy into audible sound energy. This embodiment enables a user to listen to audio playback in addition to stimulating their inner ear region in an attempt to alleviate the effects of motion sickness.

In another aspect, the present invention provides a method of stimulating the inner ear region with an electrically powered device that includes at least one inductor operatively connected to a control circuit, the method including the steps of:
locating the at least one inductor in close proximity to the inner ear region of a user; and
operating the control circuit to provide electrical energy from an electrical power source to the at least one inductor.

In one exemplary embodiment, the electrically powered device includes the necessary circuitry to allow a user to control the rate and/or amplitude of the provision of electrical energy to the at least one inductor. In this exemplary embodiment, the method of the present invention includes the step of the user adjusting the amplitude and/or frequency of the electrical energy in order to alleviate the sensation of motion sickness.

In another aspect, the present invention provides computer instruction code for execution in an electrically powered device according to the present invention, the computer instruction code including instructions to control the supply of electrical energy to the at least one inductor.

### Brief Description of the Drawings.

The present invention will now be described with the reference to the accompanying drawings, which illustrate exemplary embodiments of the present invention, wherein:
Figure 1 is a circuit diagram illustrating the main components of an embodiment of an electrically powered device according to the present invention;
Figure 2 is a flow chart illustrating the primary process steps executed by the micro-controller of the circuit arrangement as detailed in Figure 1;
Figure 3 is a diagram illustrating various waveforms detailing the pulse profile of the electrical pulses that are supplied by the control circuit of Figure 1 to an inductor;
Figure 4 is a diagrammatic representation of an exemplary embodiment of an ear attachment device that contains a control circuit and an electrical power source and includes an insertion component; and
Figure 5 is a cross sectional representation of the embodiment of Figure 4.

### Detailed Description of an Exemplary Embodiment

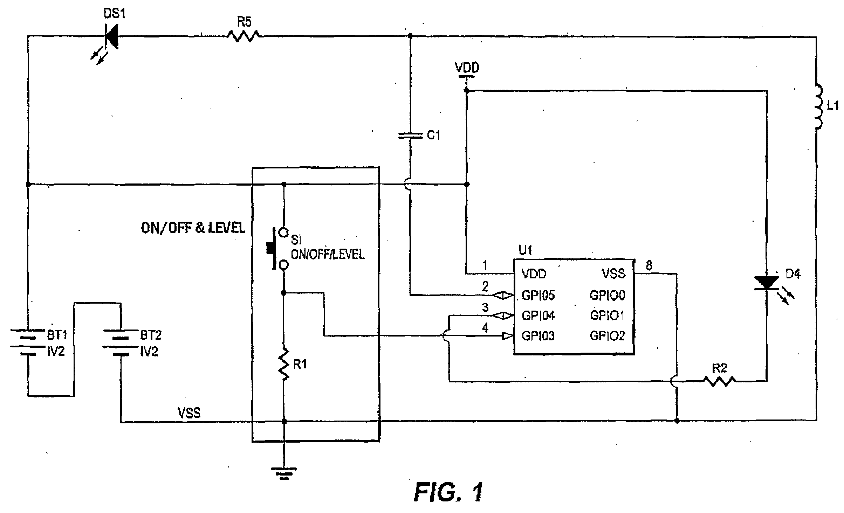

With reference to Figure 1, a circuit diagram of an exemplary embodiment of an electrically powered device according to the present invention is detailed. In the circuit arrangement of Figure 1, electrical power is supplied by the batteries BT1 and BT2 where BT1 and BT2 arc 1.2 volt batteries connected in series to provide a power source of 2.4 volts DC. The positive terminal of battery BT1 is connected to Pin 1 of the micro-controller (U1) and the negative terminal of battery BT2 is connected to Pin 8 of the micro-controller (U1) thus providing power to the micro-controller device.

The micro-controller (U1) provides a range of General Purpose Input/Output (GPIO) connections. In the instance of the exemplary embodiment of Figure 1, only three of the General Purpose Input/Output connections are used, namely, GPIO3, GPIO4 and GPIO5 with GPIO3 configured as an Input only and GPIO4 and CPIO5 configured as Output only connections.

GPIO3 is connected to the switch and resistor network formed by switch (S1) and resistor (R1). The switch (S1) is a "normally open" switch arrangement and in the normally opened condition, the resistor (R1) acts to maintain the voltage of the GPIO3 connection at the voltage Vss. Upon actuating the switching device, the switch causes electrical current to flow through resistor (R1) and a voltage of Vdd to occur at the GPIO3 connection. Accordingly, actuation of the switch S1 has the effect of causing the voltage at the GPIO3 connection to rise from Vss to Vdd for the period of time that the switch is maintained in the closed position.

The GPIO4 connection to the micro-controller (U1) is connected to the network arrangement of resistor (R2) and the light emitting diode (D4) which in turn is connected to the voltage rail Vdd. With the GPIO4 connection either at a voltage "Hi" or in a high impedance state, the light emitting diode will remain non-illuminated. However, once the GPIO4 connection is transitioned to a voltage "Lo" condition the light emitting diode (D4) will illuminate as a result of current flowing from the voltage rail Vdd through light emitting diode (D4), the resistor (R2) and ultimately through the micro-controller (U1).

The GPIO5 connection is electrically connected to a network comprising capacitor (C1) which in turn is connected to a series network of light emitting diode (DS1) and resistor (R5) and inductor (L1).

Capacitor (C1) isolates the micro-controller (U1) from the inductor and the charging circuit and provides the pulse of electrical energy to the inductor (L1). Resistor (R5) isolates the charging diode (DS1) from the inductor during normal operation and limits the current flowing through diode (DS1) during charging. Diode (DS1) rectifies the current from the inductor whilst charging and is also used to indicate when the battery is undergoing charging. Transitioning the voltage of the GPIO5 connection causes a supply of electrical energy to the inductor (L1).

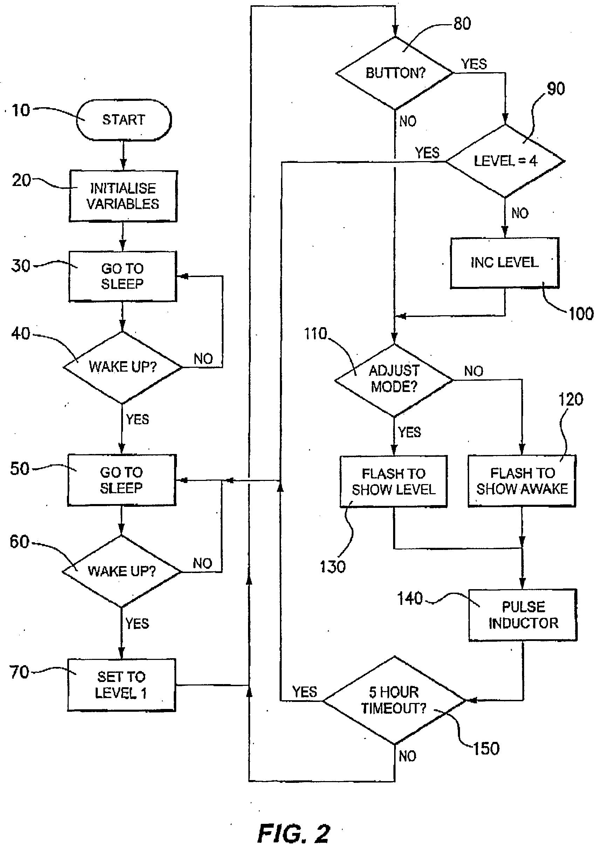

With reference to Figure 2, a flow chart detailing the main process steps executed by the micro-controller (U1) is illustrated. Upon the initial supply of power to the micro-controller (U1), the software resident in the micro-controller commences execution (Step 10). The next process executed (Step 20) is the initialisation of variables that will be used by the software as it executes individual program instructions and controls the actions of the micro-controller (U1). Having initialised the program variables, the software program proceeds to step 30 wherein it places the micro-controller (U1) into a "sleep" condition, thus minimising the electrical energy consumed by the micro-controller (U1) until the micro-controller (U1) is required to perform any necessary functions.

At Step 40, the program conducts a check to determine whether or not the micro-controller (U1) needs to "wake up" and if not, the program reverts back to Step 30.

In the event that the micro-controller (U1) detects a 'wake up" condition, the program then enters a further "go to sleep/wake up" process (steps 50/60) during which the micro-controller (U1) detects whether the user has actuated the switch. In the event that a switch actuation is detected, the program proceeds to step 70.

At Step 70, the program sets the parameters of the electrical energy pulse delivered to the inductor to the lowest level (ie. Level 1). Having set the characteristics of the electrical energy pulse to the lowest level, the micro-controller program again checks whether there has been a further actuation of the switch. In the event that a switch closure is detected, the program proceeds to Step 90 where the program then determines whether or not the current level setting of the parameters of the energy pulse are at the maximum (ie. Level 4). If not, the program proceeds to Step 100 wherein the "level" is incremented by one. At this stage, the program then proceeds to Step 110 wherein the program determines whether or not an adjustment to the mode is required. However, at Step 90, in the event that the parameters of the electrical energy pulse set to the maximum level (ie. Level 4) then the program reverts to Step 50 wherein the micro-controller (U1) reverts to the "sleep" mode.

Effectively, a manual deactivation of the device occurs if a switch closure attempts to increment the level beyond the maximum (ie. Level 4). Of course, further actuation of the switch after the program has reverted to step 50 will cause the device to "wake up" (step 60) and once again proceed to step 70 wherein an energy pulse level 1 is selected.

Referring back to Step 80 of the micro-controller program, in the event that a switch closure is not detected, the program proceeds to Step 110 wherein the program determines whether an adjustment to the mode is required.

Once the energy pulse has been incremented by a level, the program causes an LED to regularly "flash" (step 130) for approximately 30 seconds to advise the user that the level has been incremented. After 30 seconds, the program reverts to "flashing" the LED irregularly (step 120) (eg. one flash of short duration for every approximately 60 seconds). Accordingly, the purpose of step 110 is to determine whether the advisory LED needs to be flashed regularly (to indicate recent level change) or irregularly (to indicate that the device is "awake"). Irrespective of the mode, the program then proceeds to step 140 wherein a pulse of energy is delivered to the inductor thus causing the inductor to emit a pulse of electromagnetic radiation.

The program then proceeds to step 150 and in the event that 5 hours has elapsed since the last switch actuation, the micro-controller (U1) is shut down (step 50). Otherwise, the program proceeds to step 80.

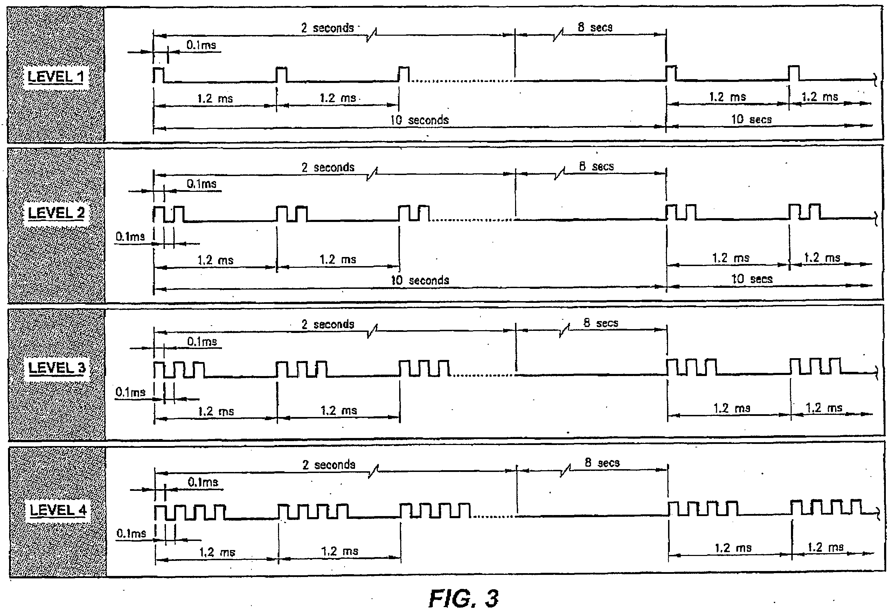

With reference to Figure 3, a diagrammatic representation of the energy pulse waveforms for each of the levels provided in the exemplary embodiment of Figures 1 and 2 are illustrated. In this particular embodiment, the micro-controller (U1) produces a series of electrical energy pulses for approximately 2 seconds. These pulses are repeated approximately every 10 seconds and the number of pulses that occur within an approximate 1.2 millisecond period depends upon the level adjustment selected by the user.

With reference to the illustrated waveform for Level 1 in Figure 3, a square wave pulse profile that lasts for a duration of approximately 0.1 milliseconds occurs for each 1.2 millisecond period. For a Level 1 selection, only a single pulse of approximately 0.1 milliseconds duration occurs for each 1.2 millisecond period and the waveform over the 1.2 millisecond period is repeated for approximately 2 seconds in duration. Upon expiry of the 2 second period, there is a delay of approximately 8 seconds before the energy pulses recommence.

With reference to the Level 2 wave form of Figure 3, it can be readily noticed that for each 1.2 millisecond period there arc 2 pulses generated that both have a duration of approximately 0.1 milliseconds with a delay of approximately 0.1 milliseconds between the pulses. Again, as for the Level 1 waveform, the energy pulses that occur over the 1.2 millisecond period are repeated for a total period of approximately 2 seconds after which a delay of approximately 8 seconds occurs before the pulses occurring within the 2 second period are repeated.

Consistent with the waveforms of Level 1 and Level 2, the reader will note that the waveforms illustrated for Level 3 and Level 4 similarly follow the framework established for Levels 1 and 2. Although, for Level 3 and Level 4, 3 and 4 pulses respectively are generated within the 1.2 millisecond period, thus providing an increased number of pulses supplied to the inductor, and hence the overall average electro-magnetic energy emitting from the inductor increases, as the user selects an increasing number of pulses within the same time period.

Following is a listing of the source code for a prototype device according to an embodiment of the invention:

```
 /************************************/
 /* Program : MotSick.C */
 /* Function : Motion Sickness device using ON/OFF switch */
 /* Language : HiTech C (ANSI C) */
 /*************************************/
 #include <pic.h>
 //#include <stdlib.h>
▪
 _CONFIG(INTIO & MCLRDIS & BOREN & PROTECT & PWRTEN &
 WDTDIS); //& WDTEN
▪
 // function prototypes
 void init(void);
 void wail_mSec(int times);
 void waist100mSec(void);
 // Defines
 #define LEDON 1
 #define LEDOFF 0
 // Action Variables
 unsigned char flash, state, test, temp;
 unsigned char speed, flashtimer, passes, inestate;
 unsigned int mSeconds;
 unsigned int timeout;
 unsigned int keytimer,count, thousands;
 unsigned char level, timer, pwm;
▪
 /**************************************/
 /* Main Program */
 /*************************************/
 main()
 {
 char i;
 for(test = 0; test<250; tcst++); // allow time during power up
 init(); // set up all port direction registers.
 //count = 0;
 //databit = 7;
 test = 0;
 timeout = 0;
 state = 0:
 passes = 0;
 level = 1;
 timer = 1;
 flashtimer = 8;
 pwm = 16;
 GIE = 1;//enable interrupts
 GPIF = 0;
 T0IE = 1;
 PIE = 1 ;
 wait_mSec(300);
 GPIF = 0;
 GPIO = 0x10; //shut off al power draining devices.
 GIE = 0; //Disable interrupts
 asm("sleep");
 asm("nop");
 for(test = 0; test<250; test++);
 GPIF = 0;
 GIE = 1; // Enable Interrupts
 T0IE = 1;
 GPIE.= 1;
 wait_mSec(200);
 speed = 20;
 incstate = 0;
 state = 0;
 level = 1;
 passes = 0;
 timeout = 0;
 for(i = 0;i<5;i++)
 GPIO5 = 0;
 GPI04 = 1;
 while(1)
      { // This is the main program endless loop.
       switch(state)
                {
                if(timeout > 1000)
                     {
                     state = 0; //Go to sleep if been on for more than 4.7 hours
                     }
                case 0: // Sleep State
                     {
                     wait_mScc(300);
                     GPIF=0;
                     GPIO = 0x10; //shut off all power draining devices.
                     GIE = 0; //Disable Interrupts
                     asm("sleep");
                     asm("nop");
                     for(test = 0; test<250; test++);
                     GPIF = 0;
                     GIE = 1; // Enable Interrupts
                     T0IE = 1;
                     GPIE = 1;
                    wait_mScc(200);
                     speed = 20;
                     incstate = 0;
                    state = 1;
                     level = 1;
                    passes = 0;
                    timeout = 0;
                     break;
                     }//end case 0:
                case 1: // Level = 1
                     {
                    flash = level;
                    if(!flashtimer)
                            {
                            GPIO0 = LEDON;
                            GPIO4 = LEDOFF; //Indicator LED ON
                            wait_mSec(20);
                            flashtimcr = speed;
                            GPIO2 = 0;
                            if(passes> 10)
                                   {
                                   flashtimer = 250;
                                   timeout++;
                                   }
                            else
                                    {
                                    passes++;
                                    }
                             break;
                             }
                     GPIO0 = LEDOFF;
                     GPIO4 = LEDON; // Indicator LED OFF
                     if(incstate) // Play Switch was Pressed
                             {
                            level = 3;
                            state = 2;
                            incstate = 0;
                            speed =10;
                            GPIO0 = LEDOFF;
                            GPIO4 = LEDON; // Indicator LED OFF
                            passes = 0;
                            }
                    break;
                    }//end case 1:
               case 2: // Level = 2
                     {
                    flash = level;
                    if(!flashtimer)
                            {
                            GPIO0 = LEDON;
                            GPIO4 = LEDOFF;
                           wait_mSec(20);
                           flashtimer = speed;
                           if(passes > 20)
                                   {
                                   flashtimer = 250;
                                   timeout++;
                                   }
                           else
                                   {
                                  passes++;
                                   }
                           break;
                           }
                   GPIO0 = LEDOFF;
                   GPIO4 = LEDON;
                   if(incstate) //Play Switch was Pressed
                           {
                           level = 5;
                          state = 3;
                          incstate = 0;
                          speed = 5;
                          GPIO0 = LEDOFF;
                             GPIO4 = LEDON;
                            passes = 0;
                            }
                     break;
                     }//end case 2:
                case 3: //Level = 4
                     {
                     flash = level;
                     if(!flashtimer)
                             {
                            GPIO0 = LEDON;
                            GPIO4 = LEDOFF;
                            wait_mSec(20);
                            flashtimer = speed;
                            if(passes > 40)
                                    {
                                   flashtimer = 250;
                                   timeout++;
                                   }
                            else
                                    {
                                   passes++;
                                    }
                            break;
                            }
                     GPIO0 =LEDOFF;
                     GPIO4 = LEDON;
                     if(incstate) // Play Switch was Pressed
                            {
                            level = 8;
                            state = 4;
                            incstate = 0;
                            speed = 2;
                            GPIO0 = LEDOFF;
                            GPIO4 = LEDON;
                            passes = 0;
                            }
                    break;
                    }//end case 3:
               case 4: //Level = 8
                    {
                    flash = level;
                    jf(!flashtimer)
                           {
                            GPIO0 = LEDON;
                           GPIO4 = LEDOFF;
                           wait_mSec(20);
                           flashtimer = speed;
                           if(passes > 80)
                                   {
                                   flashtimer = 250;
                                   timeout++;
                                   }
                           else
                                   {
                                   passes++;
                            break;
                            }
                    GPIO0 = LEDOFF;
                    GPIO4 = LEDON;
                    if(incstatc) // Play Switch was Pressed
                            {
                            level = 0;
                            state = 0;
                            incstate = 0;
                            speed = 20;
                            GPIO0 = LEDOFF;
                            GPIO4 = LEDON;
                            passes = 0;
                            }
                    break;
                    }//end case 4:
               }//end switch(state)
       if(timer < 20)
                {
               if(timer = 0)
                    {
                    timer = 100;
                    }
               if(--pwm < level)
                    {
                    GPIO5 ^= 1; //Toggle output
                    }
               else
                    {
                    GPIO5 = 0; //Output off
                    }
               if(pwm==0)
                    {
                    pwm = 16;
                    }
               }
       } // end of while endless loop
 }// end of main
 /**************************************/
 /* A milliSecond delay function */
 /**************************************/
  void wait_mSec(int times)
 {
 m Seconds = times:
 while(mSeconds);
 }//end wait_mSec();
 /***************************************/
 /* A 100 milliSecond delay function */
 /***************************************/
 void waist100mSec(void)
 {
 int times = 1000;
 while(times-);
 }//end waist100mSec();
 /***************************************/
 /* Interrupt service function *l
 /***************************************/
 static void interrupt isr(void)
 {
 //work out source of interrupt
 if(T0IF)
       { // Was this a timer ZERO overflow? Here every 100us.
       TMR0 = -86; // set for 40 Khz -15 = 25us, -40 = 50us, -90 = 100us
      temp++;
      if(temp>9)
               {
               mSeconds--;
               temp = 0;
               }//end temp>9
      if(thousands--== 0)
               {
               thousands = 1000; // here once every 100 milliseconds;
               if(timer)
                    {
                    timer--;
                    }
               if(flashtimer) flashtimer-;
               }
      T0IF = 0; // Clear interrupt flag, ready for next
 }
 if(GPIF)
       { //.Here whenever a port change interrupt occurs.
      if(GPIO3) // START Switch Down
               {//check for correct edge due to interrupt on change.
               waist100mSec(); //Debounce
               if(GPIO3)
                    {
                    while(GPIO3);
                    waist100mSec();
                    while(GPIO3);
                    // Move to next level state
                    incstate = 1;
                    passes = 0;
                    flashtimer = 1;
                    }
               GPIO =0x10;
               //Do a dummy write to clear the mismatch after lnt-on-change
               } // end of if(GPIO3)
      GPIF = 0;
 }// end if GPIF
 }// end or ISR
 void init(void)
 {
     #asm
              call 0x3FF //Load Factory Calibration Value Into OSCCAL
              bsf_STATUS,5 //Select BANKI
              movwf_OSCCAL
     #endasm
 //12f675 setup
 // ANSEL = 0; //PIC12F675 Only
     TRISIO = 0b00001110; //GP0-Output, GP1-Input,GP2-Input,GP3-
 nput,GP4-Output,GP5-Output
     GPIO0 = 0;
     GPIO4 = 1;
        VRCON = 0; //Turn Off Vrcf
        CMCON = 0x07; //Turn Off Comparator
        OPTION = 0b10001000; //Pull Ups Disabled, Rising Edge, Prescaler 1:1
 WDT
 // 76543210
        TICON = 0b00000000; // Timer 1 disabled //101; // Timer 1 enabled with
 int oscillator.
       INTCON = OxA8; // GIE-enabled, TMR0 int enabled, GP2/int disabled,
 Port change int enabled.
 // 76543210
        IOCB = 0b00001000; // Select Interrupt on change pins. Gp3 int
        PIEI = 0x00; // Disables peripheral interrupts.
 }
```

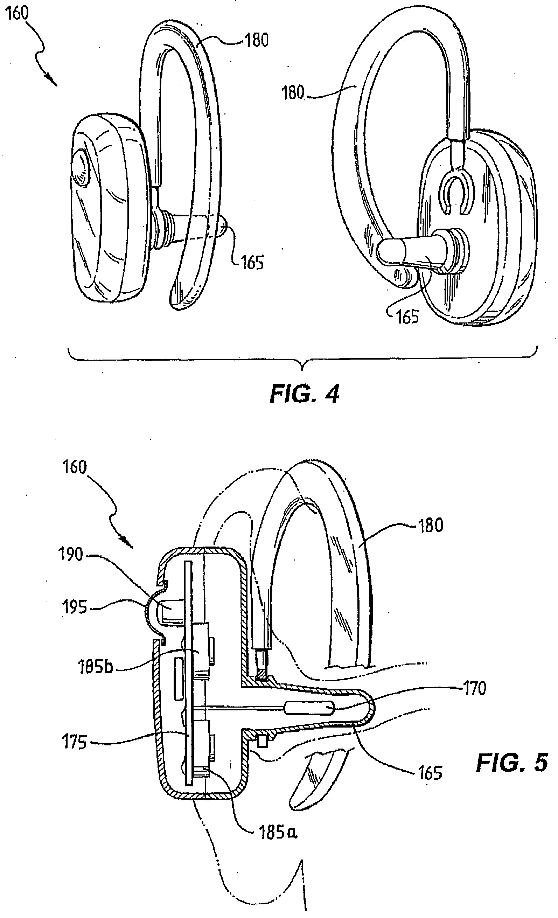

With reference to Figures 4 and 5, a diagrammatic representation of an exemplary embodiment of an ear attachment device is provided. In the specific embodiment of Figures 4 and 5, the electrically powered device (160) includes an insertion component (165) that is inserted into the car cavity of a user. The insertion component (160) contains an inductor (170) that is connected to a control circuit mounted upon a printed circuit board (175). All of the components are encapsulated within a plastic housing (that may include some flexible rubber or silicon based components) to which an ear hook (180) is removably attached to further secure the attachment of the device (160) to the car of a user.

In the embodiment of Figures 4 and 5, the electrically powered device (160) is powered by batteries (185a, 185b) that arc mounted upon the printed circuit board (175). The device (160) includes a switch (190) also mounted on the printed circuit board (175) to enable the user to activate and deactivate the device and to select differing levels of stimulation for the inner ear. A flexible rubber switch cover (195) allows easy operation of the switch (190) to select various functions.

Whilst the embodiment represented in Figures 4 and 5 is a unitary (self contained) device, other embodiments may include a separate remote control device such that the user may more easily control the operation of the device and may view the remote control device whilst operating same. For example, a remote control device may communicate control signals with the electrically powered device by a number of remote communication means such as infrared or radio frequency link. Further, rather than containing the electrically powered device in a unitary apparatus that attaches to the users' ear, the insertion component containing the at least one inductor may be connected to the control circuitry by an extended conducting lead similar to the arrangement for earphones connected to audio playback devices. Similarly, the insertion component would be connected by an extended lead to the control circuitry that may be worn or mounted in a similar manner as the various arrangements that are common for audio play back devices (e.g. belt mounted, pouch mounted or worn in pocket of article of clothing).

In one particular embodiment, the insertion component for insertion into the ear cavity of a user contains the at least one inductor and an audio play back transducer for reproducing audio signals in the car of a user. Further, the extended lead attachment to the combined insertion component/audio play back apparatus is connected to a device that provides electrical energy to the at least one inductor and audio signals to the audio transducer by respective conductors. In this particular embodiment, the user may combine the play back of audio with electro-magnetic stimulation of their inner ear such that motion sickness may be alleviated whilst at the same time not preventing the user from listening to audio signals from an audio play back device. Preferably, in this particular embodiment, a single device is used to play back audio signals and provide the electrical energy to the at least one inductor. In this embodiment, the device would allow the user to control both the electrical energy provided to the at least one inductor and all the necessary signals to provide play back of audio signals.

Those skilled in the relevant field of technology will appreciate that the invention described herein is susceptible to variations and modifications other than those specifically described. It is understood that the invention includes all such variations and modifications that fall within the spirit and scope of the present invention.

For example, although the exemplary embodiment described includes four "levels" of stimulation, it would be readily appreciated that any number of levels of stimulation may be provided. Similarly, although only one specific waveform of electrical energy supply to the at least one inductor is described in the exemplary embodiment, it would also be readily appreciated that alternative waveforms may provide greater alleviation of the sensation of motion sickness for different users. Accordingly, embodiments of the invention may include a range of different waveform stimulation programs in addition to a range of different levels for selection by a user to accommodate their personal level of discomfort.

## Claims

1. An electrically powered device including an insertion component for insertion into the ear cavity of a user, the device including at least one inductor operatively connected to a control circuit, the control circuit providing electrical energy to the at least one inductor wherein the insertion component includes the at least one inductor such that when in use, the at least one inductor is in close proximity to the inner ear of the user.

2. An electrically powered device according to claim 1 wherein the control circuit controls supply of electrical energy to the at least one inductor such that electrical energy is supplied in pulses.

3. An electrically powered device according to either claim 1 or claim 2 wherein the control circuit includes an adjustment means operable by a user, the adjustment means controlling the supply of electrical energy to the at least one inductor.

4. An electrically powered device according to claim 3 wherein the adjustment means controls the amplitude of the electrical energy supplied to the at least one inductor.

5. An electrically powered device according to either claim 3 or claim 4 wherein the adjustment means is operable to control the frequency of the supply of electrical energy to the at least one inductor.

6. An electrically powered device according to any one of claims 3, 4 or 5, wherein the adjustment means is included in a remote control device and communicates control signals by infrared or radio frequency communication.

7. An electrically powered device according to any one of the preceding claims including a micro-controller computing device operable to execute computer instruction code.

8. An electrically powered device according to claim 7 when dependent upon any one of claims 3 to 6, wherein the adjustment means is operable to select a pre-programmed profile for the supply of electrical energy to the at least one inductor, the pre-programmed profile residing in stored computer instruction code.

9. An electrically powered device according to claim 8 wherein the preprogrammed profile residing in the stored computer instruction code includes the necessary detail regarding the amplitude and/or frequency of the supply of electrical energy to the at least one inductor.

10. An electrically powered device according to any one of the preceding claims wherein the control circuit is supplied with electrical energy from batteries.

11. An electrically powered device according to claim 10 wherein the batteries are rechargeable.

12. An electrically powered device according to any one of the preceding claims wherein the insertion component includes an audio transducer for converting electrical energy into audible sound energy.

13. An electrically powered device according to claim 12 wherein the at least one inductor included in the insertion component and the audio transducer are electrically connected to a control circuit an audio signal generating the circuit respectively by extended conducting leads.

14. A method of stimulating the inner ear region with an electrically powered device that includes at least one inductor operably connected to a control circuit, the method including the steps of:
locating the at least one inductor in close proximity to the inner ear region of the user; and
operating the control circuit to provide electrical energy from an electrical power source to the at least one inductor.

15. A method according to claim 14 wherein the control circuit is operated to adjust the amplitude and/or frequency of electrical energy provided to the at least one inductor.

16. Computer instruction code for execution in an electrically powered device according to any one of claims 7 to 9, including computer instructions to control the supply of electrical energy to the at least one inductor.

17. Computer instruction code according to claim 16 wherein the instructions control the amplitude and/or frequency of electrical energy supplied to the at least one inductor.
